# EUROPEAN PATENT APPLICATION

(11) **EP 4 534 673 A1**
(43) Date of publication of application: **09.04.2025**
(21) Application number: 23816064.2
(22) Date of filing: 30.05.2023
(51) Int. Cl.: C12N 15/31, C12N 1/15, C12N 15/63, C12P 13/12, C12P 17/10

(54) **NUCLEIC ACID MODIFIED TO OBTAIN ENHANCED ERGOTHIONEINE PRODUCTIVITY, AND MICROORGANISM HAVING GENETIC MODIFICATION**

(30) Priority: 31.05.2022 JP 2022089120; 13.07.2022 JP 2022112588
(71) Applicant: Kikkoman Corporation, Noda-shi, Chiba 278-8601 (JP)
(72) Inventor: TAJIMA, Ryoko, Noda-shi, Chiba 278-8601 (JP); ICHIKAWA, Keiichi, Noda-shi, Chiba 278-8601 (JP); ITO, Kotaro, Noda-shi, Chiba 278-8601 (JP)
(74) Representative: Gill Jennings & Every LLP
(86) International application number: PCT/JP2023/020131
(87) International publication number: WO 2023/234307

(57) **Abstract**

The purpose of the present invention is to enhance ergothioneine productivity in ergothioneine production through culturing of an ergothioneine-producing filamentous fungus. In the present invention, a genetic mutation that enhances ergothioneine production in an ergothioneine-producing filamentous fungus is identified. The present invention provides: a nucleic acid that includes a conserved region at positions 433-440 in SEQ ID NO. 1 and that has, in a sequence corresponding to SEQ ID NO. 1, a deletion of a region included in a functional domain corresponding to a functional domain at W404-Q515 in SEQ ID NO. 1; and a filamentous fungus having said nucleic acid.

## Description

### FIELD

The present disclosure relates to nucleic acid modified so as to enhance productivity of ergothioneine (hereunder also referred to as "ERG"), and to a microorganism genetically modified so as to enhance productivity of ERG. It further relates to a method for producing ERG using the genetically modified microorganism.

### BACKGROUND

Ergothioneine was discovered as a sulfur-containing amino acid isolated from rye ergot fungus (*Claviceps purpurea*), and it has also been found to be present in plant and animal bodies. Plants and animals are incapable of synthesizing ergothioneine, however, and therefore ergothioneine found in plant or animal bodies is thought to derive from ergothioneine synthesized by microorganisms such as basidiomycetes. Ergothioneine is found in some basidiomycetes mushrooms, particularly edible mushrooms such as hiratake, shiitake, maitake and eringi, and is known to be especially abundant in tamogitake. One known biosynthetic pathway for ergothioneine is from histidine, with a sulfur atom being provided from cysteine. Ergothioneine has high oxidation resistance, and has been reported to have elastase-inhibiting action and tyrosinase-inhibiting action, for which reason it is of particular interest in the field of cosmetics and foods for the purpose of skin whitening and wrinkle prevention. Since ergothioneine has also been shown to contribute to the biological oxidative defense system, there have been attempts to use it in the field of medicine as well. Ergothioneine has high thermostability and pH stability and is able to maintain its antioxidant effect even at high temperatures, and it is therefore added to foods for physiological functions as well as being used as an antioxidant in foods.

Methods for producing ergothioneine include extraction from basidiomycetes such as tamogitake, chemical synthesis and fermentation using microorganisms. Extraction from basidiomycetes such as tamogitake requires time to obtain the starting materials, making it unsuitable for mass production. While chemical synthesis is suited for mass production it requires expensive synthesis reagents, and also incurs high costs for purification (PTL 1). Effective production methods include fermentation using compound C1-assimilating bacteria and yeast (PTL 2, NPL 1) and fermentation using microorganisms that overexpress ergothioneine biosynthesis genes (PTL 3), the latter being highly useful because they allow the use of microorganism fermentation products, or extracts obtained by simple extraction procedures.

### [CITATION LIST]

### [PATENT LITERATURE]

[PTL 1] Japanese Unexamined Patent Publication No. 2006-160748
[PTL 2] International Patent Publication No. WO2016/104437
[PTL 3] International Patent Publication No. WO2017/150304

### [NON PATENT LITERATURE]

[NPL 1] PLOS One 2014 vol. 9(5)e97774

### SUMMARY

### [TECHNICAL PROBLEM]

The object of the invention is to increase productivity for ERG in ERG production by microbial culturing.

### [SOLUTION TO PROBLEM]

The present inventors have carried out avid research for the purpose of increasing ERG productivity by *Aspergillus oryzae,* and as a result have found that ergothioneine productivity is enhanced by introducing a specific gene mutation into a gene of unknown function, thereupon devising the present invention.

The present invention relates to the following:
[1] Nucleic acid coding for:
   a sequence corresponding to the sequence of SEQ ID NO: 1, comprising a conserved region within SEQ ID NO: 1, wherein the sequence has a mutation in a region within a functional domain corresponding to the functional domain of W404 to Q515 of SEQ ID NO: 1, whereby the function of the functional domain is modulated; or
   a sequence having at least 90% identity with the sequence, which enhances ergothioneine production when introduced into an ergothioneine-producing microorganism.
[2] Nucleic acid according to [1] above, wherein the conserved region is the conserved region from position 433 to position 440 of SEQ ID NO: 1.
[3] Nucleic acid according to [1] above, wherein the mutation that modulates the function of the functional domain is a deletion of a region within a functional domain corresponding to the functional domain of W404 to Q515.
[4] Nucleic acid according to [1] above, wherein the deleted region includes the position corresponding to position 428 of SEQ ID NO: 1.
[5] Nucleic acid according to [1] above, wherein the deleted region comprises a deletion of 1 to 120 amino acids.
[6] Nucleic acid according to [4] above, wherein the deleted region is a region corresponding to one or more regions selected from the group consisting of position 427 to position 429, position 427 to position 440, position 427 to position 444 and position 419 to position 444 of SEQ ID NO: 1.
[7] Nucleic acid according to [3] above, wherein the deleted region is a region corresponding to one or more regions selected from the group consisting of position 431 to position 475, position 419 to position 475 and position 404 to position 515 of SEQ ID NO: 1.
[8] Nucleic acid according to [1] above, wherein the mutation that modulates the function of a functional domain has a mutation of G at the position corresponding to position 428 and/or C at the position corresponding to position 459, of SEQ ID NO: 1.
[9] Nucleic acid according to [1] above, wherein the nucleic acid is microbial nucleic acid derived from a microorganism selected from the group consisting of *Aspergillus, Penicillium, Rasamsonia* and *Talaromyces* microorganisms.
[10] A vector including nucleic acid according to any one of [1] to [9] above.
[11] A microorganism including nucleic acid according to any one of [1] to [9] above.
[12] A method for producing ergothioneine which comprises culturing a microorganism according to [11] above.
[13] A method for producing a microorganism with enhanced ergothioneine production ability, wherein the method comprises introducing, in a sequence corresponding to the sequence of SEQ ID NO: 1, comprising a conserved region from position 433 to position 440 of SEQ ID NO: 1, a mutation that modulates the function of the functional domain, into a region within a functional domain corresponding to the functional domain of W404 to Q515 of SEQ ID NO: 1, and the microorganism is an ergothioneine-producing microorganism selected from the group consisting of *Aspergillus, Penicillium, Rasamsonia* and *Talaromyces* microorganisms.
[14] The production method according to [13] above, wherein the mutation that modulates the function of the functional domain is a deletion of a region within a functional domain corresponding to the functional domain of W404 to Q515.
[15] The production method according to [13] above, wherein the conserved region is the conserved region from position 433 to position 440 of SEQ ID NO: 1.
[16] The production method according to [14] above, wherein the deleted region includes the position corresponding to position 428 of SEQ ID NO: 1.
[17] The production method according to [14] above, wherein the deleted region comprises a deletion of 1 to 120 amino acids.
[18] The production method according to [16] above, wherein the deleted region is a region corresponding to one or more regions selected from the group consisting of position 428, position 427 to position 429, position 427 to position 440, position 427 to position 444 and position 419 to position 444 of SEQ ID NO: 1.
[19] The production method according to [14] above, wherein the deleted region is a region corresponding to one or more regions selected from the group consisting of position 431 to position 475, position 419 to position 475 and position 404 to position 515 of SEQ ID NO: 1.
[20] The production method according to [14] above, wherein the mutation that modulates the function of a functional domain has a mutation of G at the position corresponding to position 428 and/or C at the position corresponding to position 459, of SEQ ID NO: 1.

### [ADVANTAGEOUS EFFECTS OF INVENTION]

According to the invention, a microorganism having, in a region within a functional domain corresponding to the functional domain of W404 to Q515 of SEQ ID NO: 1, in a gene corresponding to AO090005000664, a mutation that modulates the function of the functional domain, is able to enhance ERG production.

### BRIEF DESCRIPTION OF DRAWINGS

Fig. 1 shows the amino acid sequence of AO090005000664 (SEQ ID NO: 1) (A) and the nucleotide sequence of AO090005000664 (SEQ ID NO: 2) (B). The conserved region of position 411 to position 467 is color-highlighted.
Fig. 2 shows a comparison of ortholog genes of *Aspergillus, Penicillium, Rasamsonia* and *Talaromyces* for the conserved region (position 411 to position 479 of SEQ ID NO: 1) in the amino acid sequence of AO090005000664.
Fig. 3 shows ERG production volume for a mutated strain having the G428S mutation and C459F mutation introduced into AO090005000664 of *Aspergillus oryzae.*
Fig. 4 shows ERG production volume in a mutated strain having a mutation corresponding to G428S of SEQ ID NO: 1 introduced into the AO090005000664 ortholog gene of *Aspergillus sojae.*
Fig. 5 shows ERG production volume in a mutated strain having a mutation corresponding to G428S (G442S) introduced into the AO090005000664 ortholog gene (An16g07990) of *Aspergillus niger.*
Fig. 6 shows the results of analyzing the amino acid sequence of AO090005000664 by BLAST. The region of position 412 to position 462 is shown enlarged.

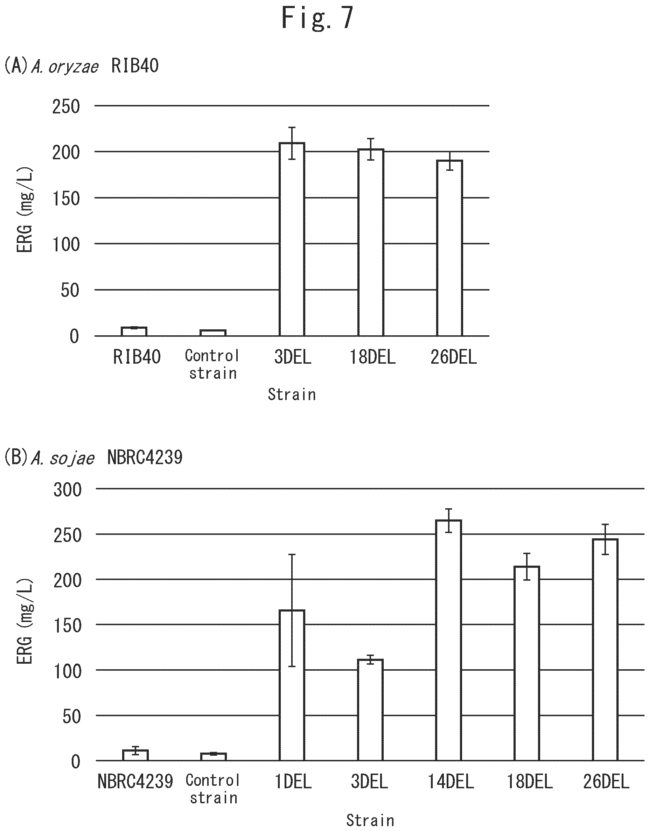

Fig. 7A-B show ERG production amounts by *Aspergillus oryzae* (A) lacking the region containing position 428 (deletion of position 427 to position 429 (3DEL), deletion of positions 427 to 444 (18DEL) and deletion of position 419 to position 444 (26DEL)) in the amino acid sequence encoded by the AO090005000664 gene, and ERG production amounts by *Aspergillus sojae* (B) lacking the region containing position 428 (position 428 (1DEL), position 427 to position 429 (3DEL), positions 427 to 440 (14DEL), positions 427 to 444 (18DEL) and position 419 to position 444 (26DEL)) in the amino acid sequence encoded by an ortholog gene having 98% identity with the AO090005000664 gene.
Fig. 7C-D show ERG production amounts by *Aspergillus oryzae* (C) having a deletion of position 431 to position 475 (45DEL), deletion of positions 419 to 475 (57DEL) and a deletion of position 404 to position 515 (112DEL) in the amino acid sequence encoded by the AO090005000664 gene.
Further shown is ERG production amounts by *Aspergillus niger* (D) having deletion of positions 441 to 443 (3DEL), positions 441 to 458 (18DEL), positions 433 to 458 (26DEL), position 433 to position 489 (57DEL) and position 418 to position 529 (112DEL) in the amino acid sequence encoded by the An16g07990 gene having 67% identity with the AO090005000664 gene.

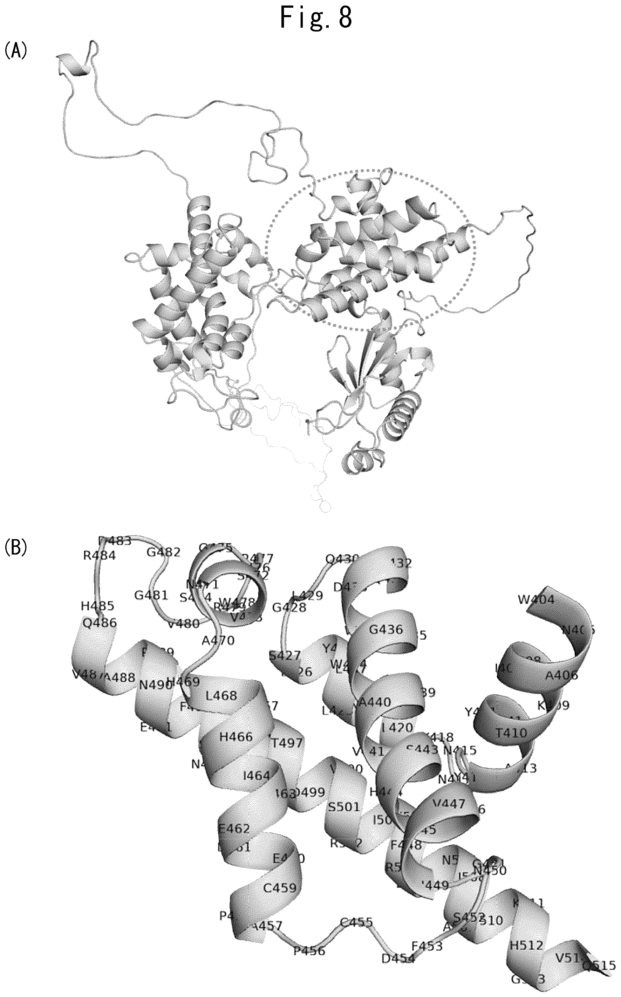

Fig. 8 shows a three-dimensional predicted structure based on homology modeling for the protein expressed by the AO090005000664 gene, using SWISS-MODEL (https://swissmodel.expasy.org/) (A). Fig. 8B shows a magnified view of the super secondary structure encircled with a dotted line (B).

### DESCRIPTION OF EMBODIMENTS

The microorganism into which the gene mutation of the invention is to be transferred may be any microorganism having ergothioneine production ability. Such microorganisms are preferably filamentous fungi, and more preferably a fungus belonging to *Aspergillus, Penicillium and Rasamsonia* and *Talaromyces* is used. Examples of filamentous fungi belonging to *Aspergillus* include *Aspergillus oryzae, Aspergillus sojae, Aspergillus luchuensis, Aspergillus tamarii, Aspergillus niger,* and *Aspergillus nidulans.* Examples of filamentous fungi strains include *Aspergillus oryzae* RIB40 and RIB326, which are publicly available strains, strains in seed koji commercially available from seed koji shops, and strains obtained by separation from food and beverage production environments such as sake and soy sauce breweries.

According to the invention, the gene with the introduced mutation is related to the gene named AO090005000664 for *Aspergillus oryzae* RIB40, and its ortholog gene. The gene has a translated region of unknown function, and consists of a nucleotide sequence (SEQ ID NO: 2) encoding a protein comprising a 657-residue amino acid sequence (SEQ ID NO: 1). The nucleotide sequence of the *Aspergillus sojae* ortholog gene of AO090005000664 gene corresponds to REGION 19840...21877 with Accession No. BaCA02000013 (SEQ ID NO: 4), and its amino acid sequence is listed as SEQ ID NO: 3. The *Aspergillus tamarii, Aspergillus luchuensis, Aspergillus niger* and *Aspergillus nidulans* ortholog genes of AO090005000664 gene are, respectively, BDV40DRAFT_264902 (amino acid sequence: SEQ ID NO: 5, nucleotide sequence: SEQ ID NO: 6), AAWM_07753 (amino acid sequence: SEQ ID NO: 7, nucleotide sequence: SEQ ID NO: 8), An16g07990 (amino acid sequence: SEQ ID NO: 9, nucleotide sequence: SEQ ID NO: 10) and ANIA_10201 (amino acid sequence: SEQ ID NO: 11, nucleotide sequence: SEQ ID NO: 12). These are highly conserved in *Aspergillus,* having amino acid sequence identity of 98% in *Aspergillus sojae,* 96% in *Aspergillus tamarii,* 67% in *Aspergillus luchuensis,* 67% in *Aspergillus niger* and 62% in *Aspergillus nidulans.* The genes have high amino acid sequence homology, at 100% in *Aspergillus sojae,* 98% in *Aspergillus tamarii*, 79% in *Aspergillus luchuensis,* 79% in *Aspergillus niger* and 75% in *Aspergillus nidulans.*

Multiple conserved regions exist in AO090005000664, which are highly conserved in other species. A particular conserved region is the region corresponding to positions 411 to 479 of SEQ ID NO: 1 (SEQ ID NO: 13). From the viewpoint of being a more highly conserved region, the conserved region is preferably the region corresponding to position 411 to position 467 (SEQ ID NO: 14), more preferably the region corresponding to position 411 to position 462 (SEQ ID NO: 15), even more preferably the region corresponding to position 411 to position 444 (SEQ ID NO: 16), yet more preferably the region corresponding to position 427 to position 444 (SEQ ID NO: 17) and even yet more preferably the region corresponding to position 433 to position 440 (SEQ ID NO: 18), of SEQ ID NO: 1. The region corresponding to position 433 to position 440 (SEQ ID NO: 18) is preferably very highly conserved in *Aspergillus,* and more preferably completely identical.

The nucleic acid of the invention may be derived from filamentous fungi such as those of *Aspergillus, Penicillium, Rasamsonia* or *Talaromyces.* The nucleic acid of the invention is highly conserved in these filamentous fungi. Nucleic acid derived from filamentous fungi belonging to *Aspergillus* encodes an amino acid sequence having 75 to 100% homology with SEQ ID NO: 1. Nucleic acid derived from filamentous fungi belonging to *Penicillium* encodes an amino acid sequence having 68 to 75% homology with SEQ ID NO: 1. Nucleic acid derived from filamentous fungi belonging to *Rasamsonia* encodes an amino acid sequence having 70% homology with SEQ ID NO: 1. Nucleic acid derived from filamentous fungi belonging to *Talaromyces* encodes an amino acid sequence having 64 to 68% homology with SEQ ID NO: 1.

According to the invention, a sequence corresponding to SEQ ID NO: 1 is the amino acid sequence of a gene which is an ortholog of AO090005000664, in a specific filamentous fungus. An ortholog gene can be selected from among the NCBI-registered protein database using the NCBI BLASTp program. Such an ortholog generally has at least 40%, at least 50% or at least 60% homology. Such a sequence corresponding to SEQ ID NO: 1 is more preferably an amino acid sequence encoded by an ortholog gene of *Aspergillus, Penicillium, Rasamsonia* or *Talaromyces.* More preferably it is an amino acid sequence of an ortholog gene of *Aspergillus oryzae, Aspergillus sojae, Aspergillus tamarii, Aspergillus luchuensis, Aspergillus niger* or *Aspergillus nidulans.* According to one mode of the invention, the sequence corresponding to SEQ ID NO: 1 can be defined by comprising a conserved region. The sequence corresponding to SEQ ID NO: 1 is therefore an amino acid sequence encoded by a gene which is an ortholog of AO090005000664, and is specified by a predetermined conserved region in SEQ ID NO: 1. Specific examples of sequences corresponding to SEQ ID NO: 1 include amino acid sequences selected from the group consisting of SEQ ID NO: 1, 3, 5, 7, 9 and 11.

An example of a conserved region is located at position 427 to position 444 of SEQ ID NO: 1 (SEQ ID NO: 17), at positions 411 to 444 (SEQ ID NO: 16), at positions 411 to 462 (SEQ ID NO: 15) or at position 433 to position 440 (SEQ ID NO: 18). From the viewpoint of matching among *Aspergillus* species, the sequence corresponding to SEQ ID NO: 1 can be defined by comprising the conserved region at position 433 to position 440 of SEQ ID NO: 1 (SEQ ID NO: 18). As alternatives to this conserved region, the conserved region within the sequence corresponding to SEQ ID NO: 1 may be defined by the following sequence: 
427 444
| |
SGLXXXXWXGXXAAXDSH (SEQ ID NO: 43)
 [where:
X430 is at least one amino acid selected from the group consisting of Q, H, P and D;
X431 is at least one amino acid selected from the group consisting of Q and L;
X432 is at least one amino acid selected from the group consisting of P, Q and G;
X433 is at least one amino acid selected from the group consisting of D and E;
X435 is at least one amino acid selected from the group consisting of R and W;
X437 is at least one amino acid selected from the group consisting of L and I;
X438 is at least one amino acid selected from the group consisting of V and M; and
X441 is at least one amino acid selected from the group consisting of V, I and L].

As another example, the conserved region is preferably a sequence which is particularly conserved in *Aspergillus.* One such conserved region is the following: 
427 444
| |
SGLXXXDWRGLVAAXDSH (SEQ ID NO: 19)
 [where:
X430 is at least one amino acid selected from the group consisting of Q, H and P;
X431 is at least one amino acid selected from the group consisting of Q and L;
X432 is at least one amino acid selected from the group consisting of P and G; and
X441 is at least one amino acid selected from the group consisting of V and I].

As yet another example, the conserved region may be the following sequence: 
411 428 444
| | |
YXAYNXXXELDXLXXXSGLXXXDWRGLVAAXDSH (SEQ ID NO: 44)
 [where:
X412 is at least one amino acid selected from the group consisting of E and D;
X416 is at least one amino acid selected from the group consisting of Q, H and E;
X417 is at least one amino acid selected from the group consisting of N, H and D;
X418 is at least one amino acid selected from the group consisting of Y, F and C;
X422 is at least one amino acid selected from the group consisting of N and S;
X424 is at least one amino acid selected from the group consisting of W, C and M;
X425 is at least one amino acid selected from the group consisting of Y, Q, S, A and G;
X426 is at least one amino acid selected from the group consisting of L, I, V and M;
X430 is at least one amino acid selected from the group consisting of Q, H and P;
X431 is at least one amino acid selected from the group consisting of Q and L;
X432 is at least one amino acid selected from the group consisting of P and G; and
X441 is at least one amino acid selected from the group consisting of V and I].

As yet another example, the conserved region may be the following sequence: 
411                    428                                        459
|                      |                                          |
YXAYNXXXELDXLXXXSGLXXXDWRGLVAAXDSHXXXXXHXXXXCXAPCEXE
  467
  |
XXXXI (SEQ ID NO: 45)
 [where:
X412 is at least one amino acid selected from the group consisting of E and D;
X416 is at least one amino acid selected from the group consisting of Q, H and E;
X417 is at least one amino acid selected from the group consisting of N, H and D;
X418 is at least one amino acid selected from the group consisting of Y, F and C;
X422 is at least one amino acid selected from the group consisting of N and S;
X424 is at least one amino acid selected from the group consisting of W, C and M;
X425 is at least one amino acid selected from the group consisting of Y, Q, S, A and G;
X426 is at least one amino acid selected from the group consisting of L, I, V and M;
X430 is at least one amino acid selected from the group consisting of Q, H and P;
X431 is at least one amino acid selected from the group consisting of Q and L;
X432 is at least one amino acid selected from the group consisting of P and G7;
X441 is at least one amino acid selected from the group consisting of V and I;
X445 is at least one amino acid selected from the group consisting of Y and F;
X446 is at least one amino acid selected from the group consisting of Q and K;
X447 is at least one amino acid selected from the group consisting of V and I;
X448 is at least one amino acid selected from the group consisting of F, V, I, Y, C and L;
X450 is at least one amino acid selected from the group consisting of N, G, D, H, E, Q and S;
X452 is at least one amino acid selected from the group consisting of S, G, D, H, A and E;
X453 is at least one amino acid selected from the group consisting of F, Y, D, C and H;
X454 is at least one amino acid selected from the group consisting of D, E, N, K and Q;
X456 is at least one amino acid selected from the group consisting of P, H, T, S and N;
X461 is at least one amino acid selected from the group consisting of D, N and S;
X464 is at least one amino acid selected from the group consisting of I, L and V;
X465 is at least one amino acid selected from the group consisting of N, D, S, C, Q, R, H and G; and
X466 is at least one amino acid selected from the group consisting of H and R].

An amino acid sequence having such a conserved region, and having at least 60% homology with respect to the amino acid sequences set forth as SEQ ID NO: 1, can be defined as a sequence corresponding to SEQ ID NO: 1. The sequence corresponding to SEQ ID NO: 1 can be specified by comprising the aforementioned conserved region, and by having at least 60%, at least 63%, at least 70%, at least 75%, at least 80%, at least 90%, at least 94%, at least 95%, at least 97%, at least 98% or at least 99% homology or identity with SEQ ID NO: 1.

According to one aspect, the invention relates to nucleic acid coding for:
a sequence corresponding to the sequence of SEQ ID NO: 1, comprising, in a region within a functional domain corresponding to the functional domain of W404 to Q515 of SEQ ID NO: 1, a mutation that modulates the function of the functional domain; or
a sequence having at least 90% identity with the sequence, which enhances ergothioneine production when introduced into an ergothioneine-producing microorganism.

According to the invention, the "functional domain" is the functional domain of W404 to Q515 of SEQ ID NO: 1, in a protein encoded by the AO090005000664 gene or its ortholog gene, or a functional domain corresponding to such a functional domain. The functional domain is indicated by the presence of a domain which adopts a super secondary structure composed of five α-helices (Figs. 8A and B). Although the specific function of this functional domain is unknown, since ergothioneine production is enhanced upon deletion at position 427 to position 429 (3DEL), a deletion at positions 427 to 444 (18DEL), and at position 419 to position 444 (26DEL), position 431 to position 475 (45DEL), position 419 to position 475 (57DEL), and position 404 to position 515 (112DEL), which are parts of the functional domain, it is clear that functional modulation of the functional domain can contribute to ergothioneine production. Considering that ergothioneine production is increased by deletion of amino acids in the functional domain, it may be that it is necessary to interfere with functioning of the functional domain in order to increase ergothioneine production. Position 428 and position 459 of SEQ ID NO: 1 are located in a continuous region of five α-helices, and mutations at those locations are highly likely to cause structural changes in the super secondary structure.

A mutation that modulates the function of a functional domain may be a mutation that affects the function of the functional domain of W404 to Q515 of SEQ ID NO: 1 or a functional domain corresponding to that functional domain, and the mutation may also have a deletion, addition or substitution of one or more amino acids of the functional domain. A "corresponding functional domain" is a functional domain of an ortholog gene of the gene designated as AO090005000664. By affecting the function of the functional domain it is possible to enhance ergothioneine productivity in an ergothioneine-producing microorganism having the AO090005000664 gene or its ortholog gene. From the viewpoint of enhancing ergothioneine production, a mutation that modulates the functional domain is a deletion in the region within a functional domain corresponding to the functional domain of W404 to Q515, or else a mutation, and particularly a substitution of G at the position corresponding to position 428 and/or C at the position corresponding to position 459.

The number of deleted amino acids is any number whereby production of ERG is enhanced in the microorganism with the deletion. As an example, the number of deleted amino acids may be a number of deletions in any range selected from the group consisting of 1 to 120, 1 to 112, 1 to 60, 1 to 57 and 1 to 47. The number of deleted amino acids may be any number of preferably 1 to 30 and even more preferably 1 to 26. Examples include deletions of amino acids in any one or more numbers selected from the group consisting of 1, 2, 3, 4, 5, 6, 7, 8, 9, 10, 11, 12, 13, 14, 15, 16, 17, 18, 19, 20, 21, 22, 23, 24, 25 and 26. More specifically, the deletion may be of the amino acid at a position corresponding to position 428, or of multiple amino acids including the amino acid at the position corresponding to position 428. According to a different aspect, the deletion may be of amino acids not including the amino acid at the position corresponding to position 428.

For example, it may be a deletion of any sequence within the conserved region from position 411 to position 462 (SEQ ID NO: 15) that includes position 428. As an example, it may be a deletion of a region corresponding to one or more regions selected from the group consisting of position 428, position 427 to position 429 and position 427 to position 440 (SEQ ID NO: 20), position 427 to position 444 (SEQ ID NO: 17), and position 419 to position 444 (SEQ ID NO: 21). As yet another example, it may be a deletion of a region corresponding to one or more regions selected from the group consisting of position 431 to position 475, position 419 to position 475 and position 404 to position 515.

According to the invention, the position of an amino acid is represented based on the amino acid position in SEQ ID NO: 1 encoded by the AO090005000664 gene, but it may also be the corresponding amino acid position in a protein encoded by a corresponding gene, i.e. an ortholog gene. The corresponding position can be determined by alignment of a homologous sequence to the amino acid sequence listed as SEQ ID NO: 1.

The identity to a sequence with deletion of a region within a functional domain corresponding to the functional domain of W404 to Q515 may be at least 60%, at least 65%, at least 70%, at least 75%, at least 80%, at least 85%, at least 90%, at least 95%, at least 97%, at least 98% or at least 99% identity. A sequence may be selected which has such identity and which increases ergothioneine productivity in a filamentous fungus when a region within a functional domain corresponding to the functional domain of W404 to Q515 has been deleted.

The function of contributing to ergothioneine production is that of carrying out some function during the course of ergothioneine production. Such a function may be one involved in transcriptional regulation of an enzyme that participates in the ergothioneine synthesis pathway or degradation pathway, or an enzyme reaction contributing to the ergothioneine synthesis pathway, or an enzyme reaction contributing to the degradation pathway of ergothioneine or its precursor. The nucleic acid of the invention coding for a sequence corresponding to the sequence of SEQ ID NO: 1, which lacks the region within a functional domain corresponding to the functional domain of W404 to Q515 of SEQ ID NO: 1, has the function of enhancing ergothioneine production by being transferred into an ergothioneine-producing filamentous fungus.

The present invention further relates to a method for producing a microorganism with enhanced ergothioneine production ability. More specifically, the production method includes deleting a region within a functional domain corresponding to the functional domain of W404 to Q515 of SEQ ID NO: 1, in a sequence corresponding to the sequence of SEQ ID NO: 1. The deleted region will usually be a region within a functional domain corresponding to the functional domain of W404 to Q515, but regions adjacent to positions 404 to 511 may also be deleted so long as ergothioneine production is enhanced. When a region included within a functional domain corresponding to the functional domain of position 404 to position 511 of SEQ ID NO: 1 is deleted, the deleted region may include or not include the position corresponding to position 428 of SEQ ID NO: 1. However, the deleted region preferably includes the position corresponding to position 428 of SEQ ID NO: 1. The deleted region is a region corresponding to one or more regions selected from the group consisting of position 428, position 427 to position 429 position 427 to position 440, position 427 to position 444 and position 419 to position 444 of SEQ ID NO: 1. The deleted region may be a deletion of the entire conserved region of position 411 to position 462 (SEQ ID NO: 15), or it may even be a deletion of the entire functional domain of position 404 to position 515. The range for the deletion may be any deletion that can enhance ergothioneine production in the microorganism having the deletion. A continuous amino acid sequence may be deleted within the functional domain of W404 to Q515, and especially within the conserved region of position 411 to position 462 (SEQ ID NO: 15), but a non-continuous amino acid sequence may also be deleted. The microorganism is an ergothioneine-producing microorganism selected from the group consisting *of Aspergillus, Penicillium, Rasamsonia* and *Talaromyces* microorganisms.

Another aspect of the invention relates to nucleic acid which has a sequence corresponding to SEQ ID NO: 1 with a mutation of G at the position corresponding to position 428 and/or C at the position corresponding to position 459 in SEQ ID NO: 1, and which enhances ergothioneine production when introduced into an ergothioneine-producing microorganism. More specifically, mutations at these positions may be a substitution of G428S and/or C459F. Nucleic acid having mutations at these positions can enhance ergothioneine production by an ergothioneine-producing microorganism. More specifically, the sequence corresponding to SEQ ID NO: 1 has a conserved region as defined herein.

The present invention further relates to nucleic acid coding for:
1) an amino acid sequence having an amino acid sequence selected from the group consisting of SEQ ID NO: 1, 3, 5, 7, 9 and 11, with S at the position corresponding to position 428 and/or F at the position corresponding to position 459, or
2) an amino acid sequence having at least 60% identity with an amino acid sequence selected from the group consisting of SEQ ID NO: 1, 3, 5, 7, 9 and 11 and with S at the position corresponding to position 428 and/or F at the position corresponding to position 459, and enhancing ergothioneine production when transferred into ergothioneine-producing filamentous fungi. Ergothioneine production can be enhanced by mutations G428S and C459F.

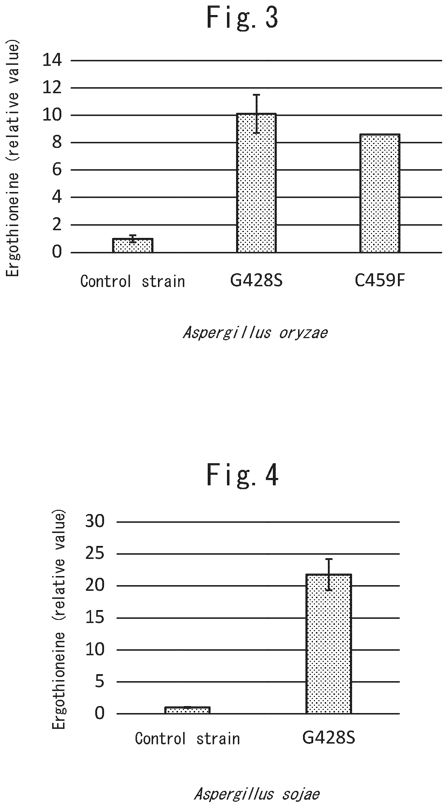

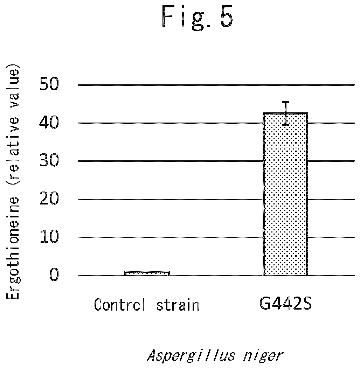

Identity with SEQ ID NO: 1, 3, 5, 7, 9 or 11 means at least 60%, at least 65%, at least 70%, at least 75%, at least 80%, at least 90% or at least 95% identity. In particular, the *Aspergillus sojae* ortholog gene and *Aspergillus niger* ortholog gene have 68% and 67% identity, respectively, and the mutation corresponding to G428S (G442S in *Aspergillus niger*) has been confirmed to enhance ERG production when transferred into *Aspergillus sojae* and *Aspergillus niger* (Figs. 4 and 5).

The present invention also relates to nucleic acid that comprises a nucleotide sequence having at least 60% identity with the nucleotide sequences of SEQ ID NO: 2, 4, 6, 8, 10 and 12 and coding for a mutation at the position corresponding to position 428 and/or the position corresponding to position 459 of the amino acid sequence listed as SEQ ID NO: 1,
and that enhances ergothioneine production when introduced into an ergothioneine-producing microorganism. Ergothioneine production can be enhanced by mutations G428S and C459F.

Identity with SEQ ID NO: 2, 4, 6, 8, 10 or 12 means at least 60%, at least 65%, at least 70%, at least 75%, at least 80%, at least 90% or at least 95% identity. In particular, the *Aspergillus sojae* ortholog gene and *Aspergillus niger* ortholog gene have 68% and 67% identity, respectively, and the mutation corresponding to G428S (G442S in *Aspergillus niger*) has been confirmed to enhance ERG production when transferred (Figs. 4 and 5).

The nucleic acid of the invention may be derived from filamentous fungi such as those of *Aspergillus, Penicillium, Rasamsonia* or *Talaromyces.* The nucleic acid of the invention is highly conserved in these filamentous fungi. An amino acid sequence encoded by nucleic acid derived from filamentous fungi belonging to *Aspergillus* has 75 to 100% homology with SEQ ID NO: 1. An amino acid sequence encoded by nucleic acid derived from filamentous fungi belonging to *Penicillium* has 68 to 75% homology with SEQ ID NO: 1. An amino acid sequence encoded by nucleic acid derived from filamentous fungi belonging to *Rasamsonia* has 70% homology with SEQ ID NO: 1. An amino acid sequence encoded by nucleic acid derived from filamentous fungi belonging to *Talaromyces* has 64 to 68% homology with SEQ ID NO: 1.

The function of contributing to ergothioneine production is that of carrying out some enzyme reaction during the course of ergothioneine production. This may be an enzyme reaction contributing to the ergothioneine synthesis pathway, or an enzyme reaction contributing to the degradation pathway of ergothioneine or its precursor. For an enzyme reaction contributing to the synthesis pathway, the protein encoded by the nucleic acid of the invention may have synthesis activity for ergothioneine or its precursor. For an enzyme reaction contributing to the degradation pathway, the protein encoded by the nucleic acid of the invention may have degradation activity for ergothioneine or its precursor or metabolite. A nucleic acid of the invention encoding an amino acid sequence having S at the position corresponding to position 428 and/or F at the position corresponding to position 459 of SEQ ID NO: 1 has the function of enhancing ergothioneine production by being transferred into an ergothioneine-producing filamentous fungus.

The position corresponding to position 428 and the position corresponding to position 459 of SEQ ID NO: 1 can be determined by alignment of a homologous sequence to the amino acid sequence listed as SEQ ID NO: 1. As one example, the position of G corresponds to position 428 in LSGLQ which is the sequence neighboring position 428, and it can be determined from the neighboring sequence. Ergothioneine production is enhanced by mutation of G to S at position 428. The position of C corresponds to position 459 in APCED, as the sequence neighboring position 459. Ergothioneine production is enhanced by mutation of C to F at position 459. The position corresponding to position 428 and the position corresponding to position 459 may have separately introduced mutations, or the mutations may be introduced simultaneously.

The present invention further relates to a method for producing a microorganism with enhanced ergothioneine production ability. More specifically, the production method comprises introducing a mutation at the position corresponding to position 428 and/or the position corresponding to position 459 in SEQ ID NO: 1, in the genome of a microorganism coding for the conserved region of the invention. The mutation at the position corresponding to position 428 is preferably G428S, and the mutation at position 459 is preferably C459F. The microorganism is an ergothioneine-producing microorganism selected from the group consisting of *Aspergillus, Penicillium, Rasamsonia* and *Talaromyces* microorganisms. As an example, the invention relates to the aforementioned production method, wherein the method comprises introduction of a mutation at the position corresponding to position 428 and/or the position corresponding to position 459, into the genome coding for the following conserved region having a mutation of G at position 428 and/or C at position 459, the conserved region corresponding to a partial sequence of SEQ ID NO: 1 (position 427 to position 444): 
427 444
| |
SGLXXXXWXGXXAAXDSH (SEQ ID NO: 43)
 [where:
X430 is at least one amino acid selected from the group consisting of Q, H, P and D;
X431 is at least one amino acid selected from the group consisting of Q and L;
X432 is at least one amino acid selected from the group consisting of P, Q and G;
X433 is at least one amino acid selected from the group consisting of D and E;
X435 is at least one amino acid selected from the group consisting of R and W;
X437 is at least one amino acid selected from the group consisting of L and I;
X438 is at least one amino acid selected from the group consisting of V and M; and
X441 is at least one amino acid selected from the group consisting of V, I and L],
and the microorganism is an ergothioneine-producing microorganism selected from the group consisting of *Aspergillus, Penicillium, Rasamsonia* and *Talaromyces* microorganisms.

Introduction of the mutations into the genome may be by any desired method of transfer. A standard transformation system may be used for transfer, or transfer may be the result of random mutation, or a genome editing technique may be used for transfer. For example, a gene cassette may be used for transfer in a transformation system, or site-specific gene transfer may be carried out, or transfer may be accomplished using a vector. Another aspect of the invention relates to a vector incorporating nucleic acid of the invention in an expressible manner. The mutation transfer method for random mutation may be, for example, treatment with ultraviolet rays (UV) or X-ray irradiation which introduces mutations by physically damaging DNA, or treatment with an alkylating reagent such as N-methyl-N'-nitro-N-nitrosoguanidine (NTG) or ethylmethanesulfonic acid (EMS) which introduces mutations by chemically damaging DNA. An example of a genome editing technique to be used is site-specific cutting and homologous recombination using a CRISPR/Cas system. The invention also relates to a microorganism comprising nucleic acid of the invention. The microorganism is one obtained as a result of the production method of the invention.

Culturing a microorganism of the invention can produce ergothioneine at a high level of productivity. The microorganism of the invention may be cultured by either solid culture or liquid culture. As an example, culturing of a microorganism of the invention allows production of ergothioneine at a high level of productivity. The microorganism of the invention may be cultured by either solid culture or liquid culture. As an example, culturing of a microorganism of the invention in steamed rice allows production of ergothioneine-containing rice malt with a high content of ergothioneine. The malt can be used to produce soy sauce, miso, salt koji or amazake by a process known in the technical field, and the *Aspergillus oryzae* can be collected to obtain mycoprotein. One aspect of the invention, therefore, relates to a method for producing rice malt with a high content of ergothioneine. The produced ergothioneine can be refined from the cultured product by an established method. As an example, the cultured product may be crushed and the water evaporated off to obtain dry powder. Ethanol-containing water may be added to the dried ergothioneine-containing material for extraction of the ergothioneine into an ethanol solution. The insoluble substances may also be removed, and the ergothioneine in the ethanol solution may be purified by chromatography.

Ergothioneine is known to have multiple effects including an antioxidant effect, an elastase inhibiting effect, a tyrosinase inhibiting effect and a polyphenol oxidase (PPO) inhibiting effect. Produced ergothioneine can therefore be purified or directly mixed with a product such as a food, cosmetic, quasi drug or drug, or prepared as a concentrate to be combined in a product. When prepared as a food, the resulting food may be a functional food. The functional indications of ergothioneine include an anti-wrinkle effect due to an elastase inhibiting effect, an effect of beautifying or whitening skin due to tyrosinase-inhibiting activity, an effect of preventing lifestyle diseases due to production of lipid peroxides, and an effect of preventing dementia or Alzheimer's disease due to elimination of active oxygen. The produced ergothioneine may be a functional food or nutritional function food, and especially a food for specified health use or a nutritional supplement, taking advantage of the physiological function of ergothioneine.

All of the publications mentioned throughout the present disclosure are incorporated herein in their entirety by reference. Homology was determined using the NCBI BLASTp program accessing the NCBI registered protein database. The percentage values for homology and identity used for the present disclosure are mass% unless otherwise specified.

The Examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

### EXAMPLES

### Example 1: Analysis of ERG high-producing strains

### (1) Medium

Eukaryotes belonging to *Aspergillus* were cultured using the following medium. For liquid culture, the following were used: Czapek-DOX (Cz-DOX) medium (3.5% Czapek-DOX broth, BD Co., pH 6.0), PD medium (2% dextrin, 1% polypeptone, 0.5% KH₂PO₄, 0.1% NaNO₃, 0.05% MgSO₄, 0.1% casamino acid, pH 6.0) and Puffmin medium (5% Puffmin SM, without pH regulation). For plate culture, the following were used: Cz-DOX agar medium (3.5% Czapek-DOX broth, BD Co., 0.1% trace element, 2% Agar addition) or Malt agar medium (4.5% Malt Agar Nissui, Nissui Pharmaceutical Co., Ltd., 0.1% trace element, 0.5% yeast extract).

### (2) Genomic analysis of ERG high-producing strains

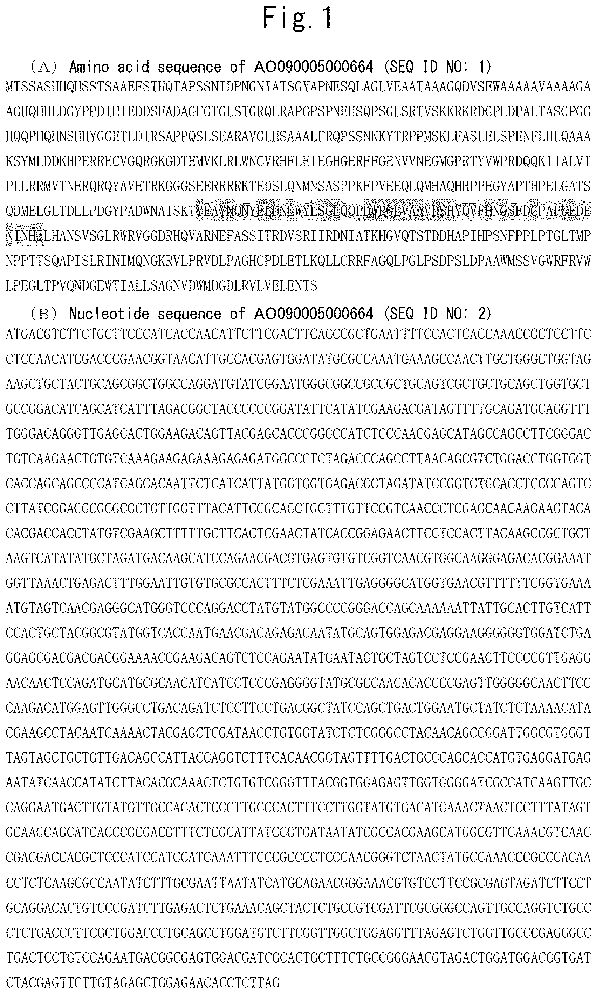

The full genomes of ERG high-producing strains were analyzed, and it was found that mutation of G428S or C459F in the AO090005000664 gene of unknown function contributes to enhanced ERG production. Fig. 1 shows the amino acid sequence (SEQ ID NO: 1) and nucleotide sequence (SEQ ID NO: 2) of AO090005000664.

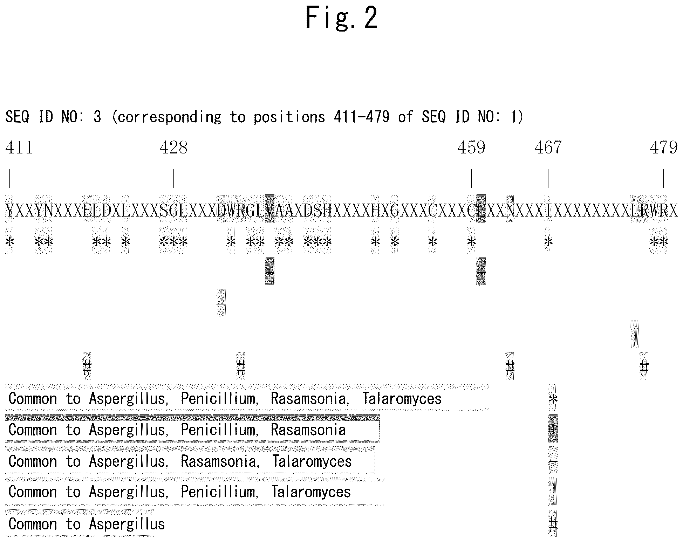

In conducting a phasicity search for the amino acid sequence of AO090005000664 using BLAST (Basic Logical Alignment Search Tool) of the NCBI (National Center for Biotechnology Information) with the default settings, a highly-conserved region was found at positions 411 to 467 of SEQ ID NO: 1, which was highly conserved in *Aspergillus* (Fig. 2). Homology between AO090005000664 and ortholog genes of *Aspergillus oryzae, Aspergillus sojae, Aspergillus tamarii, Aspergillus luchuensis, Aspergillus niger* and *Aspergillus nidulans* was found to be 100%, 98%, 96%, 67%, 67% and 62%, respectively.

### Example 2: Preparation of mutant reproduction strains of Aspergillus oryzae RIB40, and confirmation of ERG productivity

### (1) Construction of G428S cassette

A gene cassette was constructed for transfer of the G428S mutation into the AO090005000664 gene, using Aspergillus *oryzae* RIB40_dKP (Δku70, ΔpyrG) as the host. The primer sequences are shown in Table 1. A gene fragment was amplified by PCR, with the genomic sequence of RIB40 as template, using the primer sets of AoG428S_F1 and R1, AoG428S_F2 and R2, AoG428S_F3 and R3, AopyrG F and R. The PCR conditions were according to the manufacturer's protocol. The purified four PCR amplification fragments and Linearized pUC19 (Takara) (0.5 µL each) were mixed with 2.5 µL of NEBuilder HiFi DNA Assembly Master Mix (NEB), and reaction was conducted at 60°C for 1 hour. A 4 µL portion of reaction product was introduced into ECOS Competent *E*. *coli* JM109 (Nippon Gene Co., Ltd.) for transformation. The plasmids extracted from the transformants were then used as template, with the primer set AoG428S_NF and NR, and the gene fragment amplified by PCR was used as a G428S cassette. A 50 µL portion of PCR product was treated by ethanol precipitation and dissolved in 10 µL of sterilized water.

**[Table 1]**

| Table 1. Primers used to create G428S mutant reproduction strains of *Aspergillus oryzae* RIB40 | |
|---|---|
| Primer name | Sequence (5'-3') |
| AoG428S F1 | CGGTACCCGGGGATCttagatcaagtgaggaacacctectaggca (SEQ ID NO: 46) |
| AoG428S_R1 | tagcaataagcccaaCGGCCAGAGATATAGTCACTCAATTCCGTT (SEQ ID NO: 23) |
| AoG428S F2 | tcttctgagatgcagATCTCAGTGCCCGATCCGGGTAGAG (SEQ ID NO: 24) |
| AoG428S R2 | GCTGTTGTAGGCtCGAGAGATACCACAG (SEQ ID NO: 47) |
| AoG428S F3 | CTGTGGTATCTCTCGaGCCTACAACAGC (SEQ ID NO: 48) |
| AoG428S R3 | CGACTCTAGAGGATCtccgagagcaacagcgacactctca (SEQ ID NO: 25) |
| AopyrG F | ttgggcttattgctatgtccctgaaagg (SEQ ID NO: 26) |
| AopyrG R | ctgcacctcagaagaaaaggatgatcaatacc (SEQ ID NO: 27) |
| AoG428S NF | TCGGCCTCGCCTTCTTCATCGT (SEQ ID NO: 32) |
| AoG428S NR | taagtcatgtccgtaatgggcatcaaaaggg (SEQ ID NO: 33) |
| AoC459F R2 | ATTCTCATCCTCAaATGGTGCTGGGCAG (SEQ ID NO: 49) |
| AoC459F F3 | GACTGCCCAGCACCATtTGAGGATGAGAA (SEQ ID NO: 50) |

### (2) Construction of C459F cassette

A C459F cassette was constructed in the same manner as (1) above. The primers were changed from AoG428S_R2 to AoC459F_R2 and from AoG428S_F3 to AoC459F_F3.

### (3) Transformation of A. oryzae RIB40_dKP

RIB40_dKP was applied onto Cz-DOX _UraUri medium containing 10 mM uridine and 10 mM uracil, and culturing was carried out for 5 days at 30°C. After growing on a plate and scraping off the conidia with a micro loop, they were inoculated into 40 mL of PD medium containing 10 mM uridine and 10 mM uracil which had been placed in a 150 mL volume Erlenmeyer flask. Gyratory culture was carried out for 20 hours at 30°C, 180 rpm, and the cells were collected.

The cultured cells were collected with a Miracloth and washed with KCl buffer (0.6 M KCl, 0.1 M NaH₂PO₄, pH 5.5). The collected cells were suspended in 20 mL of cell-wall digesting enzyme (0.5% Lysing enzyme (Sigma-Aldrich), 0.3% Yatalase (Takara), 1.5% Cellulase Onozuka (Yakult)/20 mL KCl buffer), and after shaking for 3 hours at 30°C they were passed through a 70 µm cell strainer and the filtrate was recovered.

Following enzyme treatment, a protoplast was precipitated by centrifugation (4°C, 3000 rpm, 10 min) and the supernatant was removed. Next, 30 mL of SolB buffer (1.2 M Sorbitol, 0.1 M KCl, 10 mM Tris-HCl; pH 7.5) was added, the protoplast was resuspended and transferred to an Eppen tube, and the protoplast was recovered by centrifugation. Another 30 mL of SolB buffer was added and the same washing procedure was repeated. The recovered protoplast was suspended in 0.4 to 0.8 mL of Sol B. After then adding 5 to 10 µg of the G428S cassette or C459F cassette and 12.5 µL of SolC (50% PEG3350, 50 mM CaCl₂, 10 mM Tris-HCl; pH 7.5) to 100 µL of the protoplast suspension and slowly mixing, the mixture was incubated for 30 min on ice. A 1 mL portion of SolC was then added and mixed using a pipette, and the resulting mixture was allowed to stand at room temperature for 10 min. After then adding 10 mL of SolB, a protoplast was precipitated by centrifugation and the supernatant was removed. The total amount was then placed over regeneration agar medium (3.5% Czapek-DOX broth, 1.2 M sorbitol, 0.1% trace element, 2% agar), Top Agar (3.5% Cz-DOX broth, 1.2 M sorbitol, 0.1% trace element, 0.5% agar) at 50°C was layered over it, and culturing was carried out for about 1 week at 30°C.

The obtained transformants were spotted 3 times with Cz-DOX agar medium (3.5% Czapek-DOX broth, 0.1% trace element, 1.5% agar) for purification.

### (4) Confirming ERG production volume of mutant reproduction strains

The ERG production volumes of the obtained transformants were compared with the RIB40 wild type as a control.

### (4-1) Culturing and extraction

After preparing 10 mL of Puffmin medium (5% Puffmin SM, no pH regulation) in a 50 mL-volume Erlenmeyer flask, the cells were sterilized for 30 minutes in an autoclave at 120°C. A 100 µL portion of conidia suspension (1.0 × 10⁷/ml) was then added and shake culturing was carried out for 4 days at 30°C, 160 rpm. The culture solution was subjected to hot water extraction (90°C, 1 hour), and 0.5 mL of supernatant was filtered through a Nanosep centrifugal filtration device (3K by Nippon Pole Co., Ltd.) and appropriately diluted with ultrapure water to prepare a sample for analysis.

### (4-2) Analysis conditions

The ERG amounts in the analyzed samples were measured under the following analysis conditions.

### LC/MS analysis conditions:

Analyzer: UPLC CQ micro; Waters UPLC
Column: 2.5 HILIC 3.0 mm l.D. × 150 mm
Solvent A: 0.1% formic acid/acetonitrile
Solvent B: 0.1% formic acid/H₂O
Flow rate: 0.5 ml/min, 80% A
Inject: 3 µl
Mass spectrometer
ESI: ES+
Cone V: 21 V
Capillary V: 4.5 kV
Source temperature: 120°C
Desolvation temperature: 400°C
MS Scan Mode
LC/MS/MS ERG analysis conditions:
Analyzer: UPLC CQ micro; Waters UPLC
Column: 2.5 HILIC 3.0 mm l.D. × 150 mm
Solvent A: 0.1% formic acid/acetonitrile
Solvent B: 0.1% formic acid/H₂O
Flow rate: 0.5 ml/min, 80% A
Column washed with 100% B for each sample
Inject: 3 µL
Mass spectrometer
ESI: ES+
Cone V: 21 V
Capillary V: 4.5 kV
Source temperature: 120°C
Desolvation temperature: 400°C
Collision energy:11 V
Trace: m/z 230.1 > 186.1
Collision energy: 20 V
Trace: m/z 230.1 > 127.0

### (5) Confirmation of transformant sequences

Approximately two scrapings of the cell conidia obtained by culturing for 4 days in Cz-DOX agar medium obtained using a toothpick were suspended in 100 µL of TE, and 0.8 µL of the conidia suspension was added to 19.2 µL of KOD One (Toyobo) PCR reaction solution, for colony PCR. The PCR product was purified using a QIAquick PCR purification kit (Qiagen). Elution Buffer (Qiagen) was used for elution from the column, eluting out a volume of 50 µL as an amount equal to the PCR system. The purified PCR product was entrusted to Fasmac Co. and its sequence was confirmed.

The G428S mutation was present in 2 of the 48 G428S mutation-reproduction transformants. The C459F mutation was present in 1 of the 40 C459F mutation-reproduction transformants.

Fig. 3 shows the ERG production amounts for each strain. When the ERG productivity of the different mutated strains were compared, ERG productivity was found to be increased over the control strains and the wild strains. While ERG production was lower in the C459F mutated strain compared to the G428S mutated strain, the mutation of C459F also demonstrated that this gene mutation is involved in enhancing ERG productivity.

### Example 3: Preparation of mutant reproduction strains of Aspergillus sojae, and confirmation of ERG productivity

Mutagenesis, transformation and confirmation of ERG production volume were carried out using *Aspergillus sojae* NBRC4239_dKP (Δku70, ΔpyrG) as the host, in the same manner as the method described for preparation of AO090005000664 G428S mutant reproduction strains of *Aspergillus oryzae* RIB40 in Example 1. The ortholog gene of the AO090005000664 gene in *Aspergillus sojae* had 98% amino acid identity and 100% amino acid homology.

**[Table 2]**

| Table 2. Primer list | |
|---|---|
| Primer name | Sequence (5'→3') |
| AsG428S_F1 | CGGTACCCGGGGATCcaccgcagccaatccccagtt (SEQ ID NO: 34) |
| AsG428S_R1 | tagcaataagcccaagacatcgggaattgggtcatccgg (SEQ ID NO: 35) |
| AsG428S _F2 | tttcttctgaggtgcctcgcacagccatgctgtgcc (SEQ ID NO: 51) |
| AsG428S _R2 | GCTGTTGCAGGCtCGAGAGATACC (SEQ ID NO: 52) |
| AsgG428S_F3 | GGTATCTCTCGaGCCTGCAACAGC (SEQ ID NO: 53) |
| AsG428S_R3 | CGACTCTAGAGGATCgaccaccttggaagaaagctgacctga (SEQ ID NO: 36) |
| AopyrG_F | ttgggcttattgctatgtccctgaaagg (SEQ ID NO: 54) |
| AspyrG_R | gcacctcagaagaaaaggatgatcaatacc (SEQ ID NO: 55) |
| AsG428S_NF | gccgtgggcataggcacgattg (SEQ ID NO: 41) |
| AsG428S_NR | gctagcatcacatctcacgacacgtcc (SEQ ID NO: 42) |

The G428S mutation was present in 6 of the 19 G428S mutation-reproduction transformants.

Fig. 4 shows the ERG production amounts for each strain. When the ERG productivity of the different mutated strains were compared, ERG productivity was found to be increased over the control strains and the wild strains. The gene mutation was thus demonstrated to be involved in ERG productivity enhancement in *Aspergillus sojae* as well.

### Example 4: Preparation of mutant reproduction strains of Aspergillus niger, and confirmation of ERG productivity

Mutagenesis and transformation were carried out using *Aspergillus niger* A1179 (alternate name: MA70.15, Genotyping: kusA::amdS, cspA1 (mutation for short conidiophores), pyrG⁻) as the host, in the same manner as the method described for preparation of AO090005000664 G428S mutant reproduction strains of *Aspergillus oryzae* RIB40 in Example 1, and the ERG production volume was confirmed, with the number of culturing days changed to 6 days.

**[Table 3]**

| Table 3. Primers used for mutation transfer and creation of gene disrupted strains of *Aspergillus niger* A1179 | |
|---|---|
| Primer name | Sequence (5'→3') |
| Ang7990t_F1 | CGGTACCCGGGGATCcccgactgtgcaatatctccaattcctatc (SEQ ID NO: 56) |
| Ang7990_R1 | tagcaataagcccaaaggatggctacgatatcatatgcagtgtct (SEQ ID NO: 57) |
| Ang7990_F2 | tcttctgaggtgcggagtctgaactcgaggagcagttcaaggga (SEQ ID NO: 58) |
| Ang7990_R2 | ggctgttggaga*ctcgag*atgtaccacagg (SEQ ID NO: 59) |
| Ang7990_F3 | tgtggtacat*ctcgag*tctccaacagcc (SEQ ID NO: 60) |
| Ang7990_R3 | CGACTCTAGAGGATCaagtcgcccatgtaacgaatcaacgctaa (SEQ ID NO: 61) |
| Ang7990 Nest_F1 | cgtcagtccgcttcaatgttacc (SEQ ID NO: 62) |
| Ang7990 Nest_R1 | gttcgatgtgtatactagggttcagagtag (SEQ ID NO: 63) |
| AsPyrG2522_F | ttgggcttattgctatgtccctgaa (SEQ ID NO: 64) |
| AsPyrG4359_R | ccgcacctcagaagaaaaggatga (SEQ ID NO: 65) |

The ortholog gene of the AO090005000664 gene in *Aspergillus niger* had 67% amino acid identity and 79% amino acid homology. The mutation points in *Aspergillus oryzae* strains exhibiting ERG high productivity (G428S and C459F) are also conserved in *Aspergillus niger* (G442 and C473). Mutants were created having serine substituting for glycine at position 442 corresponding to G428S, the mutation of which has been confirmed to have an effect in *Aspergillus oryzae* RIB40. Fig. 5 shows the ERG production amounts for each strain. When the ERG productivity of the different mutated strains were compared, ERG productivity was found to be increased over the control strains and the wild strains. The gene mutation was thus demonstrated to be involved in ERG productivity enhancement in *Aspergillus niger* as well.

### Example 5: Analysis of ERG high-producing strains

### (1) Medium

Eukaryotes belonging to *Aspergillus* were cultured using the following medium. The following: Czapek-DOX (Cz-DOX) medium (3.5% Czapek-DOX broth, BD Co., pH 6.0), PD medium (2% dextrin, 1% polypeptone, 0.5% KH₂PO₄, 0.1% NaNO₃, 0.05% MgSO₄, 0.1% casamino acid, pH 6.0) and Puffmin medium (5% Puffmin SM, without pH regulation), were used for liquid culture. For plate culture, the following were used: Cz-DOX agar medium (3.5% Czapek-DOX broth, BD Co., 0.1% trace element, 2% Agar addition) or Malt agar medium (4.5% Malt Agar Nissui, Nissui Pharmaceutical Co., Ltd., 0.1% trace element, 0.5% yeast extract).

### (2) Genomic analysis of ERG high-producing strains

The full genomes of ERG high-producing strains were analyzed, and it was found that loss of amino acids in the AO090005000664 gene of unknown function contributes to enhanced ERG production. Fig. 1 shows the amino acid sequence (SEQ ID NO: 1) and nucleotide sequence (SEQ ID NO: 2) of AO090005000664.

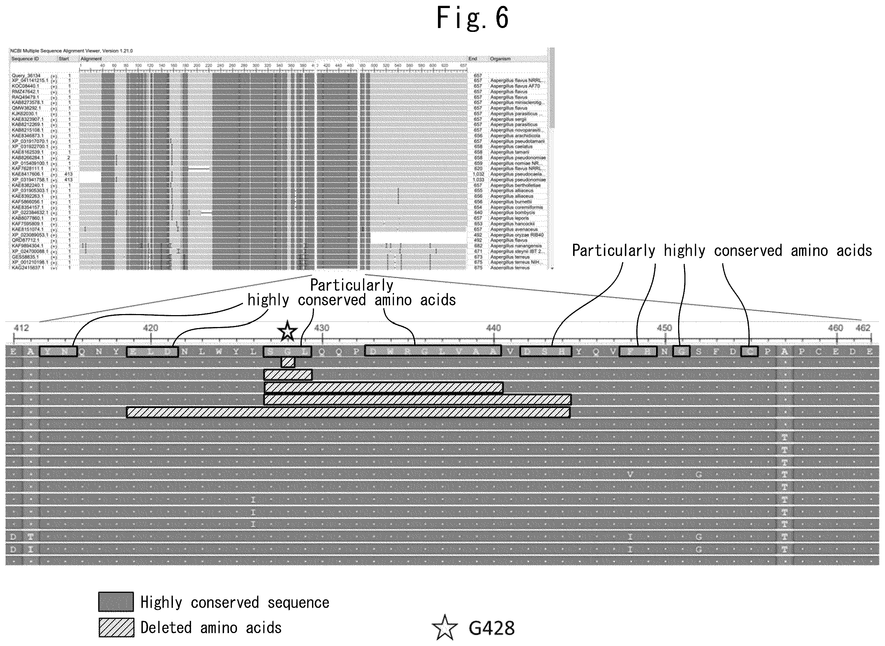

In conducting a phasicity search for the amino acid sequence of AO090005000664 using BLAST (Basic Logical Alignment Search Tool) of the NCBI (National Center for Biotechnology Information) with the default settings, a highly-conserved region was found at positions 411 to 467 of SEQ ID NO: 1, which was highly conserved in *Aspergillus* (Fig. 6). Identity between AO090005000664 and ortholog genes of *Aspergillus oryzae, Aspergillus sojae, Aspergillus tamarii, Aspergillus luchuensis, Aspergillus niger* and *Aspergillus nidulans* was found to be 100%, 98%, 96%, 67%, 67% and 62%, respectively.

### Example 6: Creation of amino acid-deficient strains of Aspergillus oryzae RIB40

### (1) Construction of amino acid-deleted cassette and obtainment of Aspergillus oryzae RIB40_dKP transformants

An amino acid-deleted gene cassette was constructed using RIB40_dKP (Δku70, ΔpyrG) as the host. The deleted amino acids were highly-conserved amino acids centered around G428 (Fig. 6). Specifically, position 428, position 427 to position 429, position 427 to position 440, position 427 to position 444 and position 419 to position 444 were each deleted. The primers used are shown in Table 1.

A gene fragment was amplified by PCR using the genomic sequence of RIB40 as template, with primer sets of primers 1 and 2, primers 3 and 4 and primers 5 and 6. The PCR conditions were according to the manufacturer's protocol. The purified three PCR amplification fragments and Linearized pUC19 (Takara) (0.5 µL each) were mixed with 2.5 µL of NEBuilder HiFi DNA Assembly Master Mix (NEB), and reaction was conducted at 60°C for 1 hour. A 4 µL portion of reaction product was introduced into ECOS Competent *E*. *coli* JM109 (Nippon Gene Co., Ltd.) for transformation. Plasmids extracted from the transformants were then used as template for amplification of gene fragments by PCR, with the sets of primers 7 and 8 (Ao3DEL), primers 9 and 8 (Ao18DEL) and primers 9 and 10 (Ao26DEL). The PCR conditions were according to the manufacturer's protocol. DpnI was added to each PCR reaction solution and reaction was conducted for 1 hour at 37°C. A 2 µl portion of each DpnI-treated PCR product was mixed with 5 µl of Ligation high Ver.2 (Toyobo), 1 µl of T4 Polynucleotide Kinase and 7 µl of sterilized water, and reaction was conducted for 1 hour at 16°C. A 5 µL portion of each reaction product was transferred into ECOS Competent *E*. *coli* JM109 (Nippon Gene Co., Ltd.) for transformation. Gene fragments amplified by PCR using each plasmid extracted from the transformants as template with a set of primers 11 and 12, were each used to prepare amino acid-deleted cassette AoXDEL cassettes (X = 3, 18, 26). A 50 µL portion of PCR product was treated by ethanol precipitation and dissolved in 10 µL of sterilized water.

The AoXDEL cassette was introduced to transform RIB40_dKP. The transformants were replanted 3 times by spotting in Cz-DOX agar medium for purification.

### (2) Conidia PCR

The cassette-introduced locations were confirmed based on band size, using primers 1 and 4. The PCR products were purified and sequenced to confirm amino acid deletion.

**[Table 4]**

| Table 4. Primers used for creation of amino acid deficient strains | | | |
|---|---|---|---|
| No. | Primer name | Sequence (5'→3') | |
| 1 | AoF1 | CGGTACCCGGGGATCttagatcaagtgaggaacacctcctaggca | (SEQ ID NO: 22) |
| 2 | AoR1 | tagcaataagoccaaCGGCCAGAGATATAGTCACTOAATTCCGTT | (SEQ ID NO: 23) |
| 3 | AoF2 | tcttetgaggtgoagATCTCAGTGCCCGATCCGGGTAGAG | (SEQ ID NO: 24) |
| 4 | AoF3 | CGACTCTAGAGGATCtccgagagcaacagcgecactctca | (SEQ ID NO: 25) |
| 5 | pyrG_F | ttgggcttattgctatgtccctgaaagg | (SEQ ID NO: 26) |
| 6 | pyrG_R | ctgcacctcagaagaaaaggatgatcaatacc | (SEQ ID NO: 27) |
| 7 | 430F | CAACAGCCGGATTGGCGTGGGTTA | (SEQ ID NO: 28) |
| 8 | 426R | GAGATACCACAGGTTATCGAGCTCGTAGTT | (SEQ ID NO: 29) |
| 9 | 445F | TACCAGGTCTTTCACAACGGTAGTTTTGAC | (SEQ ID NO: 30) |
| 10 | 418R | GTAGTTTTGATTGTAGGCTTCGTATGTTTT | (SEQ ID NO: 31) |
| 11 | AoNF | TCGGCCTCGCCTTCTTCATCGT | (SEQ ID NO: 32) |
| 12 | AoNR | taagtcatgtccgtaatgggcatcaaaaggg | (SEQ ID NO: 33) |
| 13 | AsF1 | CGGTACCCGGGGATCcaccgcagccaatccccagtt | (SEQ ID NO: 34) |
| 14 | AsR1 | tagcaataagcccaagacatcgggaattgggtcatccgg | (SEQ ID NO: 35) |
| 15 | AsR2 | CGACTCTAGAGGATCgaccaccttggaagaaagctgacctga | (SEQ IDNO: 36) |
| 16 | As429F | CTGCAACAGCCGGATTGGCGTG | (SEQ ID NO: 37) |
| 17 | 427R | CGAGAGATACCACAGGTTATCGAGCTCG | (SEQ ID NO: 38) |
| 18 | As441F | GTTGACAGCCATTACCAAGTCTTTCACAAC | (SEQ ID NO: 39) |
| 19 | As445F | TACCAAGTCTTTCACAACGGTAGTTTTGAC | (SEQ ID NO: 40) |
| 20 | AsNF | gccgtgggcataggeacgattg | (SEQ ID NO: 41) |
| 21 | AsNR | gctagcatcacatctcacgacacgtcc | (SEQ ID NO: 42) |
| 22 | Ang_F1 | CGGTACCCGGGGATCccaagccctctgggttaacatacacga | (SEQ ID NO: 66) |
| 23 | Ang _R1 | Tagcaataagcccaaaggatggctacgatatcatatgcagtgtct | (SEQ ID NO: 67) |
| 24 | Ang_F2 | Tcttctgaggtgcagcgaagaagtctgaactcgaggagcagt | (SEQ ID NO: 68) |
| 25 | Ang_R2 | CGACTCTAGAGGATCtcgcccatgtaacgaatcaacgctaa | (SEQ ID NO: 69) |
| 26 | Ang444F | Caacagcoggattggcgtggac | (SEQ ID NO: 70) |
| 27 | Ang440R | Gatgtaccacaggttgtcaagttcataatt | (SEQ ID NO: 71) |
| 28 | Ang459F | Ttccaggtcattcacaacggcgg | (SEQ IDNO: 72) |
| 29 | Ang432R | Ataattatgattgtacaggtcatacgactt | (SEQ ID NO: 73) |
| 30 | Ang_NF | Agaataagggtcacgaggtgatggtct | (SEQ ID NO: 74) |
| 31 | Ang_NR | Tgcatcatgaggtagaactttaggtgctac | (SEQ ID NO: 75) |
| 32 | 416F | TTACGGTGGAGAGTTGGTGGGGATC | (SEQ ID NO: 76) |
| 33 | 430R | TTGTAGGCCCGAGAGATACCACAGG | (SEQ ID NO: 77) |
| 34 | 516F | ACGTCAACCGACGACCACGCT | (SEQ ID NO: 78) |
| 35 | 403R | GTCAGCTGGATAGCCGTCAGGAAGG | (SEQ ID NO: 79) |

### (3) Confirmation of amino acid deleted strains and ERG production volumes

The obtained transformants and a control were cultured for 7 days at 26°C in 10 ml of 5% Puffmin SM medium in a 50 ml-volume Erlenmeyer flask. After culturing, they were heated for 1 hour at 90°C with the autoclave in lysing/incubating mode, and the supernatant was filtered with a Nanosep centrifugal filtration device (3K by Nippon Pole Co., Ltd.) and quantified by LC/MS/MS (Fig. 7A).

### (3-2) Analysis conditions

The ERG amounts in the analyzed samples were measured under the following analysis conditions.

### LC/MS analysis conditions:

Analyzer: UPLC CQ micro; Waters UPLC
Column: 2.5 HILIC 3.0 mm l.D. × 150 mm
Solvent A: 0.1% formic acid/acetonitrile
Solvent B: 0.1% formic acid/H₂O
Flow rate: 0.5 ml/min, 80% A
Inject: 3 µl
Mass spectrometer
ESI: ES+
Cone V: 21 V
Capillary V: 4.5 kV
Source temperature: 120°C
Desolvation temperature: 400°C
MS Scan Mode
LC/MS/MS ERG analysis conditions:
Analyzer: UPLC CQ micro; Waters UPLC
Column: 2.5 HILIC 3.0 mm l.D. × 150 mm
Solvent A: 0.1% formic acid/acetonitrile
Solvent B: 0.1% formic acid/H₂O
Flow rate: 0.5 ml/min, 80% A
Column washed with 100% B for each sample
Inject: 3 µL
Mass spectrometer
ESI: ES+
Cone V: 21 V
Capillary V: 4.5 kV
Source temperature: 120°C
Desolvation temperature: 400°C
Collision energy: 11 V
Trace: m/z 230.1 > 186.1
Collision energy: 20 V
Trace: m/z 230.1 > 127.0

### Example 7: Creation of amino acid-deleted strains of Aspergillus sojae NBRC4239

### (1) Construction of amino acid-deleted cassette and obtainment of Aspergillus sojae NBRC4239_dKP transformants

A gene cassette was constructed for amino acid deletion in the same manner as *Aspergillus oryzae* RIB40 described above, using NBRC4239_dKP (Δku70, ΔpyrG) as the host. A gene fragment was amplified by PCR using the genomic sequence of NBRC4239 as template, with primer sets of primers 13 and 14, primers 3 and 15 and primers 5 and 6. The PCR conditions were according to the manufacturer's protocol. The purified three PCR amplification fragments and Linearized pUC19 (Takara) (0.5 µL each) were mixed with 2.5 µL of NEBuilder HiFi DNA Assembly Master Mix (NEB), and reaction was conducted at 60°C for 1 hour. A 4 µL portion of reaction product was introduced into ECOS Competent *E*. *coli* JM109 (Nippon Gene Co., Ltd.) for transformation. Plasmids extracted from the transformants were then used as template for amplification of gene fragments by PCR, with the sets of primers 16 and 17 (As1DEL), primers 7 and 8 (As3DEL), primers 18 and 8 (As14DEL), primers 9 and 8 (As18DEL) and primers 19 and 10 (As26DEL). Gene fragments amplified by PCR using plasmids obtained in the same manner as *Aspergillus oryzae* RIB40 described above as template with a set of primers 20 and 21, were each used to prepare amino acid-deleted cassette AsXDEL cassettes (X = 1, 3, 14, 18, 26). A 50 µL portion of PCR product was treated by ethanol precipitation and dissolved in 10 µL of sterilized water.

The AsXDEL cassette was introduced to transform NBRC4239_dKP. The transformants were replanted 3 times by spotting in Cz-DOX agar medium for purification.

### (2) Conidia PCR

The cassette-introduced locations were confirmed based on band size, using primers 13 and 15. The PCR products were purified and sequenced to confirm amino acid deletion.

### (3) Confirmation of amino acid deleted strains and ERG production volumes

The ERG production volumes of amino acid-deleted strains were confirmed in the same manner as for *Aspergillus oryzae* RIB40 used in Example 2(3) (Fig. 7B).

### Example 8: Creation of amino acid-deficient strains of Aspergillus oryzae RIB40

### (1) Construction of amino acid-deleted cassette and obtainment of Aspergillus oryzae RIB40_dKP transformants

An amino acid-deleted gene cassette was constructed using RIB40_dKP (Δku70, ΔpyrG) as the host. This was used as the amino acids for deletion of all or part of the domain, based on the predicted structure in Example 10. Specifically, position 431 to position 475, position 419 to position 475 and position 404 to position 515 were each deleted. The primers used are shown in Table 1.

Gene fragments were amplified by PCR using the plasmids created in Example 2 as template, and with the sets of primers 22 and 23 (Ao45DEL), primers 22 and 10 (Ao57DEL) and primers 24 and 25 (Ao112DEL). The PCR conditions were according to the manufacturer's protocol. DpnI was added to each PCR reaction solution and reaction was conducted for 1 hour at 37°C. A 2 µl portion of each DpnI-treated PCR product was mixed with 5 µl of Ligation high Ver.2 (Toyobo), 1 µl of T4 Polynucleotide Kinase and 7 µl of sterilized water, and reaction was conducted for 1 hour at 16°C. A 5 µL portion of each reaction product was transferred into ECOS Competent E. *coli* JM109 (Nippon Gene Co., Ltd.) for transformation. Gene fragments amplified by PCR using each plasmid extracted from the transformants as template with a set of primers 11 and 12, were each used to prepare amino acid-deleted cassette AoXDEL cassettes (X = 45, 57, 112). A 50 µL portion of PCR product was treated by ethanol precipitation and dissolved in 10 µL of sterilized water.

The AoXDEL cassette was introduced to transform RIB40_dKP. The transformants were replanted 3 times by spotting in Cz-DOX agar medium for purification.

### (2) Conidia PCR

The cassette-introduced locations were confirmed based on band size, using primers 1 and 4. The PCR products were purified and sequenced to confirm amino acid deletion.

### (3) Confirmation of amino acid deleted strains and ERG production volumes

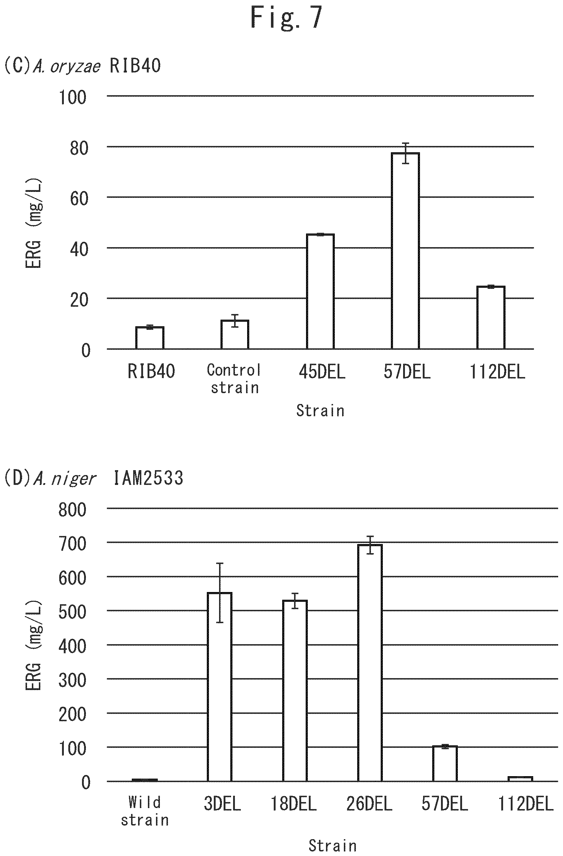

The ERG production volumes of amino acid-deleted strains were confirmed in the same manner as for *Aspergillus oryzae* RIB40 used in Example 2(3) (Fig. 7C).

### Example 9: Creation of amino acid-deleted strains of Aspergillus niger FGSC A1179

### (1) Construction of amino acid-deleted cassette and obtainment of Aspergillus niger FGSC A1179 transformants

A gene cassette was constructed for amino acid deletion in the same manner as *Aspergillus oryzae* RIB40 described above, using FGSC A1179 (ΔkusA, pyrG-) as the host. A gene fragment was amplified by PCR using the genomic sequence of *Aspergillus niger* IAM2533 as template, with primer sets of primers 26 and 27, and primers 28 and 29. The PCR conditions were according to the manufacturer's protocol. The two purified PCR amplification fragments and the purified gene fragments of purified primers 5 and 6 of Example 2 were mixed with 2.5 µL of NEBuilder HiFi DNA Assembly Master Mix (NEB) and Linearized pUC19 (Takara) (0.5 µL each), and reaction was conducted for 1 hour at 60°C. A 4 µL portion of reaction product was introduced into ECOS Competent *E*. *coli* JM109 (Nippon Gene Co., Ltd.) for transformation. Plasmids extracted from the transformants were then used as template for amplification of gene fragments by PCR, with the sets of primers 30 and 31 (Ang3DEL), primers 32 and 31 (Ang18DEL), primers 32 and 33 (Ang26DEL), primers 34 and 33 (Ang57DEL) and primers 35 and 36 (Ang112DEL). Gene fragments amplified by PCR using plasmids obtained in the same manner as *Aspergillus oryzae* RIB40 described above as template with a set of primers 37 and 38, were each used to prepare amino acid-deleted cassette AsXDEL cassettes (X = 3, 18, 26, 57, 112). A 50 µL portion of PCR product was treated by ethanol precipitation and dissolved in 10 µL of sterilized water.

The AngXDEL cassette was introduced to transform FGSC A1179. The transformants were replanted 3 times by spotting in Cz-DOX agar medium for purification.

### (2) Conidia PCR

The cassette-introduced locations were confirmed based on band size, using primers 26 and 29. The PCR products were purified and sequenced to confirm amino acid deletion.

### (3) Confirmation of amino acid deleted strains and ERG production volumes

The ERG production volumes of amino acid-deleted strains were confirmed in the same manner as for *Aspergillus oryzae* RIB40 used in Example 2(3) (Fig. 7D).

### Example 10: Structural analysis of protein expressed from AO090005000664 gene

The predicted spatial configuration for PAAG _04735 was used as a template for tertiary structure prediction for the AO090005000664 gene, using the homology modeling function of the SWISS-MODEL (https://swissmodel.expasy.org/) (Fig. 8). For the template tertiary structure, a search was made from among genes with high homology with AO090005000664, for those with known tertiary structures or with published predicted tertiary structures, and the predicted tertiary structure for PAAG_04735 (from *Paracoccidioides lutzii*) with 43% identity was selected. The predicted tertiary structure of PAAG_04735 was that predicted by AlphaFold2. As of the current time the predicted structure of OAory_01008280 which has 100% identity with AO090005000664 gene is publicly available at AlphaFold Protein Structure Database (https://alphafold.com/), and the predicted structure was found to be similar as determined by homology modeling. The predicted structure of OAory_01008280 had not yet been published at the time of homology modeling.

According to research by the present inventors, the amino acid sequence encoded by the AO090005000664 gene has deletion of position 427 to position 429 (3DEL), deletion of positions 427 to 444 (18DEL) and deletions of position 419 to position 444 (26DEL), position 431 to position 475 (45DEL), position 419 to position 475 (57DEL) and position 404 to position 515 (112DEL) (Figs. 7A and C). Position 431 to position 475 corresponded to the second α-helix and the third helix of the super secondary structure domain composed of five α-helices. Position 404 to position 515 corresponded to the entire super secondary structure domain.

It was also shown that ergothioneine production is enhanced in *A. sojae* having a gene modified so as to have a deletion at position 428 (1DEL), a deletion at position 427 to position 429 (3DEL), a deletion at positions 427 to 440 (14DEL), a deletion at positions 427 to 444 (18DEL) and a deletion at position 419 to position 444 (26DEL), from the amino acid sequence encoded by an ortholog gene with 98% identity with the AO090005000664 gene (Fig. 7B).

It was further shown that ergothioneine production is enhanced in *A. niger* having a gene modified so as to have a deletion at position 441 to position 443 (3DEL), a deletion at position 441 to position 458 (18DEL), a deletion at position 433 to position 458 (26DEL), a deletion at position 433 to position 489 (57DEL) and a deletion at position 418 to position 529 (112DEL), from the An16g07990 gene with only 67% identity with the AO090005000664 gene (Fig. 7D).

While the function of the super secondary structure domain included within W404 to Q515 including position G428 in the AO090005000664 gene and its corresponding genes is still unknown, it was shown that ergothioneine productivity can be increased by at least partially inhibiting the function of this super secondary structure domain. It will be readily understood by a person having ordinary skill in the art that the positions mentioned in the AO090005000664 gene will be different corresponding positions for different genes.

### [Sequence Listing]

C:\Users\syutsugan-03\Documents\P-boy\HTML\P230228WO_20230530_ 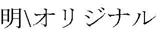 \P230228WO.xml

## Claims

1. Nucleic acid coding for:
a sequence corresponding to the sequence of SEQ ID NO: 1, comprising a conserved region within SEQ ID NO: 1, wherein the sequence has a mutation in a region within a functional domain corresponding to the functional domain of W404 to Q515 of SEQ ID NO: 1, whereby the function of the functional domain is modulated; or
a sequence having at least 90% identity with said sequence,
wherein the nucleic acid enhances ergothioneine production when introduced into an ergothioneine-producing microorganism.

2. The nucleic acid according to claim 1, wherein the conserved region is the conserved region from position 433 to position 440 of SEQ ID NO: 1.

3. Nucleic acid according to claim 1, wherein the mutation that modulates the function of the functional domain is a deletion of a region within a functional domain corresponding to the functional domain of W404 to Q515.

4. Nucleic acid according to claim 1, wherein the deleted region includes the position corresponding to position 428 of SEQ ID NO: 1.

5. Nucleic acid according to claim 1, wherein the deleted region comprises a deletion of 1 to 120 amino acids.

6. Nucleic acid according to claim 4, wherein the deleted region is a region corresponding to one or more regions selected from the group consisting of position 427 to position 429, position 427 to position 440, position 427 to position 444 and position 419 to position 444 of SEQ ID NO: 1.

7. Nucleic acid according to claim 3, wherein the deleted region is a region corresponding to one or more regions selected from the group consisting of position 431 to position 475, position 419 to position 475 and position 404 to position 515 of SEQ ID NO: 1.

8. Nucleic acid according to claim 1, wherein the mutation that modulates the function of a functional domain has a mutation of G at the position corresponding to position 428 and/or C at the position corresponding to position 459, of SEQ ID NO: 1.

9. Nucleic acid according to claim 1, wherein the nucleic acid is microbial nucleic acid derived from a microorganism selected from the group consisting of *Aspergillus, Penicillium, Rasamsonia* and *Talaromyces* microorganisms.

10. A vector including nucleic acid according to any one of claims 1 to 9.

11. A microorganism including nucleic acid according to any one of claims 1 to 9.

12. A method for producing ergothioneine which comprises culturing a microorganism according to claim 11.

13. A method for producing a microorganism with enhanced ergothioneine production ability, wherein the method comprises introducing in a sequence corresponding to the sequence of SEQ ID NO: 1 comprising the conserved region from position 433 to position 440 of SEQ ID NO: 1, a mutation that modulates the function of the functional domain into a region within a functional domain corresponding to the functional domain of W404 to Q515 of SEQ ID NO: 1, and the microorganism is an ergothioneine-producing microorganism selected from the group consisting of *Aspergillus, Penicillium, Rasamsonia* and *Talaromyces* microorganisms.

14. The production method according to claim 13, wherein the mutation that modulates the function of the functional domain is a deletion of a region within a functional domain corresponding to the functional domain of W404 to Q515.

15. The production method according to claim 13, wherein the conserved region is the conserved region from position 433 to position 440 of SEQ ID NO: 1.

16. The production method according to claim 14, wherein the deleted region includes the position corresponding to position 428 of SEQ ID NO: 1.

17. The production method according to claim 14, wherein the deleted region comprises a deletion of 1 to 120 amino acids.

18. The production method according to claim 16, wherein the deleted region is a region corresponding to one or more regions selected from the group consisting of position 428, position 427 to position 429, position 427 to position 440, position 427 to position 444 and position 419 to position 444 of SEQ ID NO: 1.

19. The production method according to claim 14, wherein the deleted region is a region corresponding to one or more regions selected from the group consisting of position 431 to position 475, position 419 to position 475 and position 404 to position 515 of SEQ ID NO: 1.

20. The production method according to claim 14, wherein the mutation that modulates the function of a functional domain has a mutation of G at the position corresponding to position 428 and/or C at the position corresponding to position 459, of SEQ ID NO: 1.
